**Europäisches Patentamt**

**European Patent Office**

**Office européen des brevets**

(19)

(11) Veröffentlichungsnummer: **0 243 984**
**A2**

(12) ## EUROPÄISCHE PATENTANMELDUNG

(21) Anmeldenummer: 87107366.4

(22) Anmeldetag: 04.07.84

(51) Int. Cl.⁴: **A61M 25/00**

(30) Priorität: **15.07.83 DE 3325650**

(43) Veröffentlichungstag der Anmeldung:
**04.11.87 Patentblatt 87/45**

(60) Veröffentlichungsnummer der früheren
Anmeldung nach Art. 76 EPÜ: **0 131 847**

(84) Benannte Vertragsstaaten:
**DE FR GB**

(71) Anmelder: **MED Inventio AG**
**Seestrasse 359**
**CH-8038 Zürich-Wollishofen(CH)**

(72) Erfinder: **Frimberger, Eckart, Dr.**
**Tristanstrasse 12**
**D-8000 München 40(DE)**

(74) Vertreter: **Körber, Wolfhart, Dr.**
**Patentanwälte Dipl.-Ing. H. Mitscherlich**
**Dipl.-Ing. K. Gunschmann Dr.rer.nat. W.**
**Körber Dipl.Ing. J. Schmidt-Evers Dipl.-Ing.**
**W. Melzer Steinsdorfstrasse 10**
**D-8000 München 22(DE)**

(54) **Spanneinrichtung für eine Versteifungssonde.**

(57) Es wird eine Spanneinrichtung (10) für eine Versteifungssonde (1) beschrieben, die zur Verwendung in der Medizin beispielsweise zu therapeutischen oder diagnostischen Zwecken, insbesondere zur Einführung in den Verdauungs-oder Ausscheidungstrakt vorgesehen ist. In die Spanneinrichtung (10) werden die gleichsinnigen Enden des Mantels (2) und der Seele (3) der Versteifungssonde eingehängt. Mit Hilfe von Gegendruckfedern (17, 18) und durch mittels einem Gewindebolzen (20) verschiebbaren Druckstücken (15, 16) wird die Seele (3) relativ zum Mantel (2) verspannt.

FIG.3

## Spanneinrichtung für eine Versteifungssonde

Die Erfindung bezieht sich auf eine Versteifungssonde zur Verwendung in der Medizin zu therapeutischen oder diagnostischen Zwecken, insbesondere zur Einführung in den Verdauungs-oder Ausscheidungstrakt. Sie bezieht sich auch auf eine Spanneinrichtung zum Betätigen der Versteifungssonde.

Verschiedene Eingriffe in der Medizin können nur unter Zuhilfenahme von sogenannten Versteifungsdrähten durchgeführt werden. Dies trifft beispielsweise zu auf die Plazierung von Schläuchen in den Zwölffingerdarm, zur Ernährung oder zum Absaugen von Flüssigkeit zur Diagnostik. Ein peroral über die Speiseröhre in den Magen vorgeschobener weicher Schlauch bildet beim weiteren Vorschieben im Magen zahlreiche Schlingen, die es unmöglich machen, den Schlauch aus dem Magen hinaus in den Zwölffingerdarm vorzuschieben. Wenn in den Schlauch ein rigider Draht eingeführt wird, kann der Magen ohne wesentliche Schlingenbildung passiert werden. Nachdem der Zwölffingerdarm erreicht ist, wird der Versteifungsdraht aus dem Schlauch entfernt.
Ähnliche Probleme gibt es bei der Plazierung von Speiseröhren-Prothesen und Prothesen im Gallengangbereich. Diese bekannten Versteifungsdrähte weisen jedoch den Nachteil auf, daß sie eine vorgegebene Rigidität aufweisen, was beim Passieren von Organen, wo beispielsweise ein weicherer Draht oder wiederum ein härterer Draht günstiger wäre, sich unvorteilhaft auswirkt.

Aufgabe der Erfindung ist es, einen Versteifungsdraht (Versteifungssonde) anzugeben, dessen Rigidität kontinuierlich und kontrollierbar entsprechend den Bedürfnissen in einfacher Weise verändert werden kann.

Diese Aufgabe wird durch die im Anspruch 1 enthaltenen Merkmale gelöst.

Der wesentliche Vorteil der Erfindung besteht darin, daß die Rigidität der Sonde wahlweise vergrößert werden kann, und zwar mit einfachen und kleinbauenden Mitteln, nämlich durch das an sich bekannte Prinzip des Bowdenzugs, wobei die bei axialem Zug an der Seele sich einstellende Versteifung des Bowdenzugs zunutze gemacht wird, die bei einem üblichen Bowdenzug an sich ohne Bedeutung ist, weil es bei einem Bowdenzug im wesentlichen darauf ankommt, längs wirksame Kräfte zu übertragen. Erfindungsgemäß ist das Einführende der Seele durch ein Anschlagteil am Mantel abgestützt. Die Zugkraft wird somit dem Mantel unmittelbar übertragen. Hierdurch kommt eine bei sonstigen Bowdenzügen übliche Abstützung dieses Bowdenzugendes in Fortfall, so daß das Einführende für die zweckbestimmten Handhabungen frei ist. Um die fallweise erforderliche Versteifungs-Spannung zu erzeugen, ist der Sonde gemäß Anspruch 13 eine Spanneinrichtung zugeordnet, die mit einem verstellbaren Glied an der Seele angreift und in der das ihr zugewandte Ende des Mantels gehalten ist.

Für eine befriedigende Funktion bedarf es eines Mantels, der quer zu seiner Längsrichtung biegsam und in seiner Längsrichtung nicht oder nur unwesentlich komprimierbar ist, so daß die angestrebte Versteifung erreicht werden kann. Dies kann durch einen flexiblen Mantel bestimmter Grundsteifigkeit und auch durch die Ausgestaltungen nach den Ansprüchen 2 und 3 erreicht werden. Bei der Ausgestaltung nach Anspruch 3 können die Flächen, mit denen die Glieder aneinanderliegen, zueinander parallel oder auch schräg verlaufen. Im ersten Fall wird die Sonde beim Versteifen bestrebt sein, sich zu begradigen. Im zweiten Fall sind von einer Gerade abweichende Versteifungsformen der Sonde möglich, z.B. eine Krümmung. Wenn die Flächen, mit denen die Glieder der Sonde aneinanderliegen, gemäß Anspruch 4 sphärische Flächen sind, läßt sich die Sonde in ihrer jeweiligen Form versteifen, was insbesondere beim Einführen in empfindliche Körperteile von Bedeutung ist. Bei allen vorbeschriebenen Ausgestaltungen ist beispielsweise der Vorteil gegeben, daß beim Einführen eines Schlauches in den Zwölffingerdarm die als Einführ-Hilfe verwendete Versteifungssonde ein verhältnismäßig angenehmes perorales Einschieben über die Speiseröhre in den Magen erlaubt. Beim Eintreten des vorderen Schlauchendes in den Magen, kann nun die Versteifungssonde entsprechend rigidisiert werden, so daß der Schlauch problemlos in den Zwölffingerdarm vorgeschoben werden kann. Nach Erreichen des Zwölffingerdarms wird die Versteifungssonde wieder entspannt, und sie kann ohne unangenehme Erscheinungen für den Patienten aus dem Schlauch entfernt werden.

Die Ausgestaltungen nach Anspruch 5 empfehlen sich , um eine Spanneinrichtung einfach und - schnell an die Sonde anzusetzen bzw. zu entfernen.

Das oder die Anschlagteile können je nach Bedarf die Form einer Kugel oder einer senkrecht mit ihrer Stirnfläche zur Zugrichtung angeordnete Scheibe aufweisen (Anspruch 6), sie können aber auch jede andere, zu therapeutischen o.a. Zwecken oder zum festen Spannen in der Spanneinrichtung vorteilhafte Form aufweisen.

Gemäß Anspruch 7 können die Anschlagteile unlösbar durch Kleben, Schweißen o.ä. befestigt sein. Sie können aber auch beispielsweise über ein Schraubgewinde lösbar befestigt sein. Letzterer Fall weist den Vorfall auf, daß nach Ablösen des Anschlagteils die Seele aus dem Mantel herausgezogen und somit beide Teile wirksamer gereinigt und desinfiziert werden können. Des weiteren kann ein lösbar befestigtes Anschlagteil durch ein Anschlagteil anderer Form ersetzt werden, so daß die Versteifungssonde den unterschiedlichsten Verwendungsmöglichkeiten in der Medizin zugedacht werden kann.

Es ist von Vorteil, wenn beide aus dem Mantel hervorragende Enden der Seele des Bowdenzugs je ein Anschlagteil oder am einen Ende ein Anschlag-oder Befestigungsteil und am anderen Ende ein zu therapeutischen oder diagnostischen Zwecken aktives Teil aufweisen.

Durch die Ausgestaltung nach Anspruch 9 kann die Entnahme von festen Proben ermöglicht werden, beispielsweise eines Gallensteins, oder diese Ausgestaltung kann sogar zum Zerquetschen eines Gallen-oder Blasensteins benutzt werden. Der Korb ist dabei derart geformt, daß zur Aufnahme des Steines das aus dem Mantel hervorgeschobene Seelenende den Korb sich radial erweitern läßt. Durch Zurückziehen der Seele wird der Korb das Bestreben zeigen, seine längs gerichteten Drähte radial einwärts zu drücken, und somit den Korb völlig zusammengelegt in das Mantelinnere hineinzuzwängen. Durch diese radiale Einwärts-Druckbewegung kann ein in den Korb aufgenommener Stein verklemmt und aus dem Körper entfernt oder sogar derart zerquetscht werden, daß die feinen Steinfragmente aus dem Organ ausgeschieden werden können.

Insbesondere wenn der Mantel kein vollvolumig einstückiger Körper ist, sondern aus Wicklungen oder Gliedern besteht, kann eine verhältnismäßig glatte Oberfläche durch die im Anspruch 1o enthaltenen Ausgestaltungsmerkmale erreicht werden.

So können Verunreinigungen vermieden und die Sauberhaltung des Gerätes besser vollzogen werden. Auch kann ein entsprechender Kunststoff-oder sonst in der Medizin gebräuchlicher Überzug auf allen Oberflächen der erfindungsgemäßen Sonde vorgesehen sein.

Des weiteren kann die erfindungsgemäße Sonde an dem aus dem Mantel hervorragenden vorderen Ende ein über das Anschlagteil herausragendes weiches Führungsende aufweisen, was das Einführen der Sonde erleichtert.

Dem gleichen Zweck dient auch die Ausgestaltung nach Anspruch 11, wobei gleichzeitig ein leichtes und bequemes Anschließen einer Spanneinrichtung oder ein leichtes und bequemes Bewegungsblockieren von Seele und Mantel ermöglicht ist.

Im Anspruch 12 sind Ausgestaltungsmerkmale enthalten, die eine Bewegungsblockierung zwischen einer Sonde und einem darauf aufzuschiebenden Schubglied ermöglichen. Hierzu kann vorteilhaft ein entweder auf dem Schubglied oder auf der Sonde befestigtes Klemmteil oder Kupplungsteil dienen, das mit einem jeweils am anderen Teil befestigten Kupplungsteil zusammenwirkt. Durch diese Ausgestaltung wird die Handhabung der Sonde beim Einführen einer Prothese oder dergleichen erheblich vereinfacht.

Die im Anspruch 14 enthaltene Spanneinrichtung zeichnet sich nicht nur durch eine einfache Bauweise aus, sondern sie ermöglicht auch eine einfache Handhabung beim Versteifen der Sonde. Soll die Sonde versteift werden, dann wird das ein Anschlagteil oder Befestigungsteil aufweisende Ende der Seele in die Halterung des zweiten Druckstückes eingelegt und von Hand am Mantel des Bowdenzugs entgegen der Kraft der kleinen, weichen Feder gezogen, bis sich das Mantelende in die am ersten Querträger vorgesehene Halterung einbringen läßt. Danach wird der Gewindebolzen durch Drehen aus dem zweiten Druckstück in Richtung des ersten Druckstücks hin verschoben, bis die Stirnfläche des Gewindebolzens an der Stirnfläche des ersten Druckstücks unter nur leichter Spannung ansteht. Hierdurch kann nun die zum Einführen benötigte Vorspannung erreicht sein, die eine ausreichende Weichheit der Sonde ergibt. Durch weiteres Vorschieben des Gewindebolzens wird der Abstand zwischen dem ersten Querträger und dem zweiten Druckstück entgegen der Kraft der kräftigeren Feder vergrößert, wobei durch das gleichzeitige Herausziehen der Seele die Sonde ihre gewünschte Steifigkeit erhält.

Die Ausbildung nach Anspruch 15 zeichnet durch Einfachheit bei guter Funktion aus. Die Führungsstangen dienen gleichzeitig zur Aufnahme und Führung der Druckfedern und stellen zudem die Längsseiten des Rahmens der Einrichtung dar.

Die Ausgestaltungen nach den Ansprüchen 16 bis 19 dienen der Halterung des dem Einführende abgewandten Ende des Mantels, wobei der Schlitz die Einführung und die Aufweitung die Aufnahme des Mantels ermöglicht. Diese Aufweitung kann konisch ausgebildet sein, wodurch das in sie eingeführte Mantelende hier verklemmt wird. Sie kann aber auch eine zylindrische Bohrung sein, die einen etwas größeren Druchmesser als der Außendurchmesser des Mantels besitzt. Die Stirnfläche des eingeführten Mantels stützt sich am Bohrungsgrund ab, während die Mantelfläche des eingeführten Endes in der zylindrischen Bohrung gleichzeitig eine Führung bzw. radiale Abstützung erfährt. Hierdurch wird zudem ein Abknicken des Bowdenzugs verhindert. Die im Anspruch 19 enthaltenen Merkmale ermöglichen den Anschluß der Spanneinrichtung an Sonden mit unterschiedlichen Durchmessern bzw. Abmessungen ihrer Mäntel bzw. Seelen. In einem solchen Fall ist es vorteilhaft, die ersten Halterungen in wenigstens einem Träger anzuordnen, der soweit beweglich an der Spanneinrichtung gehalten ist, daß die ersten Halterungen jeweils in eine bestimmte Arbeitsposition bringbar sind. Eine vorteilhafte Ausführungsform für eine solche Lösung ist z.B. ein Revolverkopf.

Die gleichen Vorteile gelten auch für die Ausführungsformen nach den Ansprüchen 21 und 22. In den Schlitz der zweiten Halterung wird die Seele derart eingelegt, daß das Anschlagteil an der der ersten Halterung entgegengesetzten Stirnfläche der zweiten Halterung ansteht. Besonders vorteilhaft ist es, wenn diese zweite Halterung eine von der ersten Halterung weggeschwungene halbkreisförmige Ausbildung aufweist, wodurch eine sichere Befestigung des Anschlagteiles, insbesondere bei dessen kugelförmiger Ausbildung, sichergestellt ist. Dabei kann diese Halterung aus einem Blech geformt sein, welches C-förmig gebogen und in dessen Bogenteil der Schlitz eingebracht ist. Durch den Anspruch 22 wird es ermöglicht, zum einen unterschiedlich große oder unterschiedlich geformte Sonden in mehrere nebeneinanderliegende zweite Halterungen einzusetzen, wobei in diesem Fall die zweiten Halterungen unterschiedliche Formen aufweisen, z.B. unterschiedlich breite Schlitze. Zum anderen ist es möglich, im Sinne der Ausgestaltung nach Anspruch 19 eine oder mehrere zweite Halterungen so verschiebbar anzuordnen, daß sie in eine bestimmte Arbeitsposition bringbar sind. Wenn mehrere zweite Halterungen in Längsrichtung hintereinander angeordnet sind, ist es möglich, unterschiedlich lange Sonden aufzunehmen bzw. unterschiedlichen Positionen der Seele gegenüber dem Mantel Rechnung zu tragen.

Um Sonden unterschiedlicher Dimensionen bzw. Formen verwenden zu können, ist es auch möglich die Halterungen lösbar anzubringen, so daß beispielsweise bei Verwendung einer dickeren Sonde eine entsprechend dimensionierte Halterung ausgetauscht werden kann. Hierdurch können durch Bereitstellen einer einzigen Einrichtung und unter Verwendung mehrerer austauschbarer Halterungen auch Sonden unterschiedlicher Dimensionen gespannt werden, was die Wirtschaftlichkeit dieser Einrichtung wesentlich erhöht. Auf der nach innen zeigenden Weise der Oberfläche mindestens eines der Seitenteile oder des Rahmens kann in vorteilhafterweise eine maßbandähnliche Markierung in bekannter Weise aufgetragen werden. Zudem kann auf dem ersten Druckstück ein quer angeordneter Markierungsstrich angebracht werden, welcher im Zusammenwirken mit der vorgenannten Markierung das Spannungsmaß der Versteifungssonde anzeigt (Ansprüche 24 und 25).

Der Anspruch 27 bezieht sich darauf, die Spanneinrichtung als Arretierungseinrichtung für die Sonde zu verwenden. Hierfür ist am zweiten Druckstück eine zusätzliche Klemmeinrichtung, wie Klemmschraube u.ä., vorzusehen, welche das zweite Druckstück auf den Führungselementen festklemmt. Hierdurch kann das zweite Druckstück in vorbestimmtem Abstand im Verhältnis zum ersten Querträger arretiert werden, wodurch die Länge der aus dem Mantel hervorragenden Seelenteile festgelegt werden kann.

Nachfolgend wird die Erfindung anhand mehrere Ausführungsbeispiele der Versteifungssonde und der Spanneinrichtung anhand der Zeichnung näher beschrieben.

Es zeigt:

Fig. 1 eine Draufsicht auf eine Versteifungssonde nach der Erfindung mit einem kugelförmigen und einem scheibenförmigen Anschlagteil;

Fig. 2 ein Ende einer erfindungsgemäßen Versteifungssonde mit einem korbförmigen Anschlagteil;

Fig. 3 eine Draufsicht auf eine Spanneinrichtung nach der Erfindung mit einem abgenommenen Seitenteil;

Fig. 4 einen axialen Schnitt nach den Linien IV-IV aus Fig. 3;

Fig. 5 eine Ansicht nach dem Pfeil III aus Fig. 3;

Fig. 6 einen Schnitt nach den Linien IV-IV in Fig. 1;

Fig. 7 - 1o jeweils Draufsichten auf die erfindungsgemäße Spanneinrichtung in unterschiedlichen Betätigungsstadien;

Fig. 11 eine erfindungsgemäß ausgestaltete Spanneinrichtung als zweites Ausführungsbeispiel;

Fig. 12 eine Ansicht auf die Spanneinrichtung nach Fig 11 in Richtung des Pfeiles XII;

Fig. 13 eine erfindungsgemäß ausgestaltete Sonde als drittes Ausführungsbeispiel;

Fig. 14 eine Einzelheit des dritten Ausführungsbeispiels;

Fig 15 eine erfindungsgemäß ausgestaltete Sonde als viertes Ausführungsbeispiel;

Fig. 16 eine erfindungsgemäß ausgestaltete Sonde als fünftes Ausführungsbeipiel;

Fig. 17 eine Sonde als sechstes Ausführungsbeispiel.

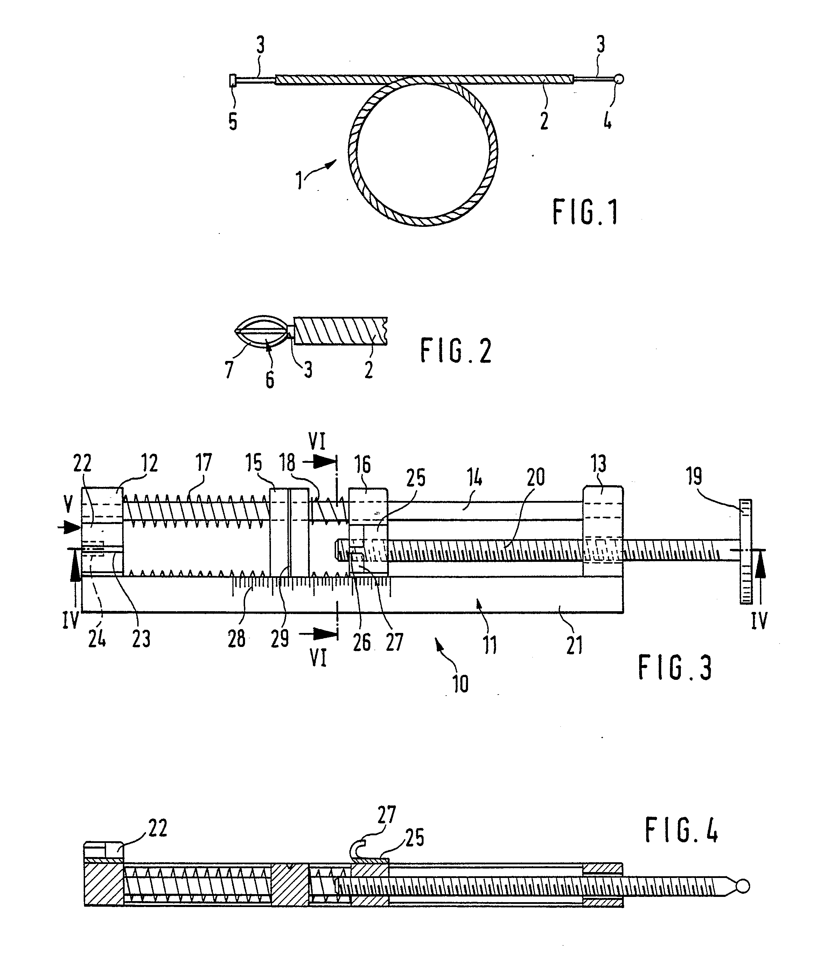

Wie aus Fig. 1 zu entnehmen ist, besteht die erfindungsgemäße Versteifungssonde aus einem spiralförmigen Mantel 2 und einer in diesem axial verschieblich angeordneten weichen Seele 3. An den beiden aus dem Mantel 2 hervorragenden Enden der Seele 3 sind jeweils Anschläge befestigt, und zwar beispielhaft in dieser Ausführung ein kugelförmiger Anschlagteil 4 und am anderen Ende ein scheibenförmiger Anschlag 5.

Wie aus Fig.2 zu ersehen ist, kann der Anschlagteil auch ein aus längsgerichteten elastischen Federstahldrähten 7 geformten Korb 6 sein.

Wie Fig.3 und 4 zeigen, besitzt die Spanneinrichtung 10 einen ersten und einen zweiten - schmalen Querträger 12 bzw. 13, die an ihren Enden über Führungsstäbe 14 zu einem rechteckigen Rahmen 11 fest miteinander verbunden sind.

Zwischen den Querträgern 12 und 13 sind auf den Führungsstäben 14 gleitend bzw. axial beweglich ein erstes und ein zweites Druckstück 15 bzw. 16 angeordnet. Zwischen dem ersten Querträger 12 und dem ersten Druckstück 15 ist eine lange, harte Druckfeder 17 und zwischen dem ersten Druckstück 15 und dem zweiten Druckstück 17 eine weiche, kurze Druckfeder 18 jeweils auf den Führungsstangen 14 axial geführt angeordnet.

Ein ein Griffteil 19 aufweisender Gewindebolzen 20 ist mittig derart angeordnet, daß er den zweiten Querträger 13 durch eine glatte Führungsbohrung durchdringt, während er in dem zweiten Druckstück 16 über eine Gewindebohrung eingeschraubt ist.

Zu beiden Längsseiten sind an dem Rahmen 11 U-förmige Seitenteile 21 lösbar befestigt, die zur kompakten und geschlossenen Bauweise der Einrichtung beitragen. In der Darstellung nach Fig.3 ist das eine Seitenteil 21 zwecks besserer Veranschaulichung des Zusammenbaues der Einrichtung entfernt worden.

Auf dem ersten Querträger 12 ist eine erste Halterung 22 über Schrauben oder dgl. lösbar befestigt. An der Oberseite der Halterung 22 ist ein axialer Schlitz 23 vorgesehen, welcher an seiner nach außen weisenden Seite eine zylindrische Ausweitung 24 in Form einer Bohrung aufweist.

Auf der Oberseite des zweiten Druckstückes 16 ist eine zweite Halterung 25 über Schrauben oder dgl. lösbar befestigt. Auch diese Halterung weist einen nach oben hin offenen Schlitz 26 zum Einlegen der Seele 3 auf. Die Halterung 25 weist eine hakenförmige Ausbildung auf, wobei der gerade Teil zur Befestigung auf dem Druckstück und der gekrümmte Hakenteil 27 zur Befestigung des Anschlagteiles 4 dient.

Auf der Oberseite des einen Seitenteiles 21 ist eine maßbandähnliche Markierung 28 in bekannter Weise aufgetragen, während das erste Druckstück 15 eine Markierung 29 besitzt.

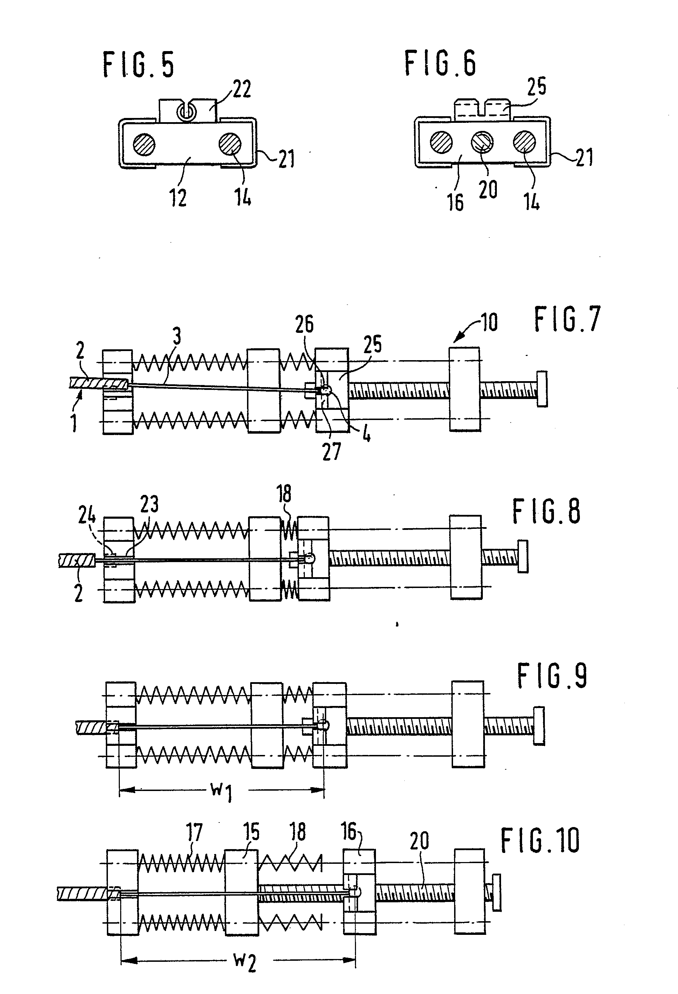

Wie aus den Fig.7 bis 10 zu erkennen ist, wird zum Spannen der Versteifungssonde deren eines Ende in die Spanneinrichtung 10 eingelegt. Und zwar wird das herausragende Ende der Seele 3 in der Haltevorrichtung 25 derart eingelegt, daß die Seele durch den Schlitz 26 geschoben wird und der Anschlagteil 4 sich in dem Hakenteil 27 festhängt (siehe Fig.7). Dann wird von Hand in Längsrichtung der Einrichtung gezogen, bis das Mantelteil 2 die Seele 3 in einer Länge freigibt, die ein Einlegen der Seele in den Schlitz 23 erlaubt. Hierbei wird das Druckstück 16 entgegen der Kraft der Feder 18 in Richtung auf das im wesentlichen durch diese leichte Zugkraft unbeweglich stehende feste Druckstück 15 bewegt. Nach erfolgter Einlage der Seele 3 in den Schlitz 23 wird der Mantel 2 losgelassen, so daß dieser in die Ausweitung 24 unter Kraft der Feder 18 hineinrutscht. Hierdurch wird ein nur leicht vorgespannter Zustand der Versteifungssonde 1 erreicht. Die Versteifungssonde 1 weist die gewünschte Flexibilität zur Einführung beispielsweise über die Speiseröhre in den Magen auf.

Soll nun bei Erreichen des Magens die Versteifungssonde eine höhere Steifigkeit aufweisen, wird, wie dies aus Fig.10 ersichtlich ist, der Gewindebolzen 20 mit Hilfe seines Griffteiles 19 in der Gewindebohrung des zweiten Druckstükkes 16 eingeschraubt, bis die Stirnseite des Gewindebol zens 20 an der ihr zugekehrten Stirnseite des ersten Druckstückes 15 ansteht. Danach wird der Gewindebolzen in gleicher Richtung weiter eingeschraubt, wodurch der Abstand zwischen den beiden Druckstücken 15 und 16 vergrößert wird. Dadurch daß die Feder 17 eine relativ harte Feder ist, wird das Druckstück 15 diese Feder 17 nur in geringem Maße zusammendrücken, während durch Vergrößerung des Abstandes zwischen den beiden Druckstücken 15 und 16 der Abstand zwischen der Stirnseite des Mantels und der Anlagefläche des Anschlagteiles 4 vergrößert wird. Die hierdurch entstehende große Spannkraft zwischen Seele und Mantel verursacht eine hohe Steifigkeit der Versteifungssonde 1.

Der als Versteifungssonde 1 verwendete Baudenzug muß bezüglich der Längen des Mantels und der Seele als auch die Einrichtung bezüglich der wirksamen Abstände $w_1$ und $w_2$ in der Dimemsionierung aufeinander abgestimmt sein. Auch müssen die Federn 17 und 18 ihren Federweg und Federkennlinie entsprechend den erforderlichen Zug-bzw. Spannkräften angepaßt sein.

Die erfindungsgemäße Spanneinrichtung kann auch in anderer, nicht dargestellter Weise aufgebaut werden, indem die Anordnung der Federn und der Druckelemente in anderer Weise zueinander angeordnet werden.

Eine einfachere, jedoch nicht so optimal wirksame Ausführungsform kann dadurch erzielt werden, daß nur das Druckstück 15 vorgesehen wird, welches über einen in dem festen Querteil 13 eingeschraubten Gewindebolzen direkt gegen eine Druckfeder 17 gedrückt wird. In dem eingedrückten Zustand kann die Versteifungssonde in eine auf der Oberseite des Druckstückes 15 vorgesehene Halteeinrichtung 25 eingehängt und in der Halteeinrichtung 22 eingelegt werden. Durch Ausschrauben und damit Entspannen der Feder 17 kann dann der Baudenzug versteift werden.

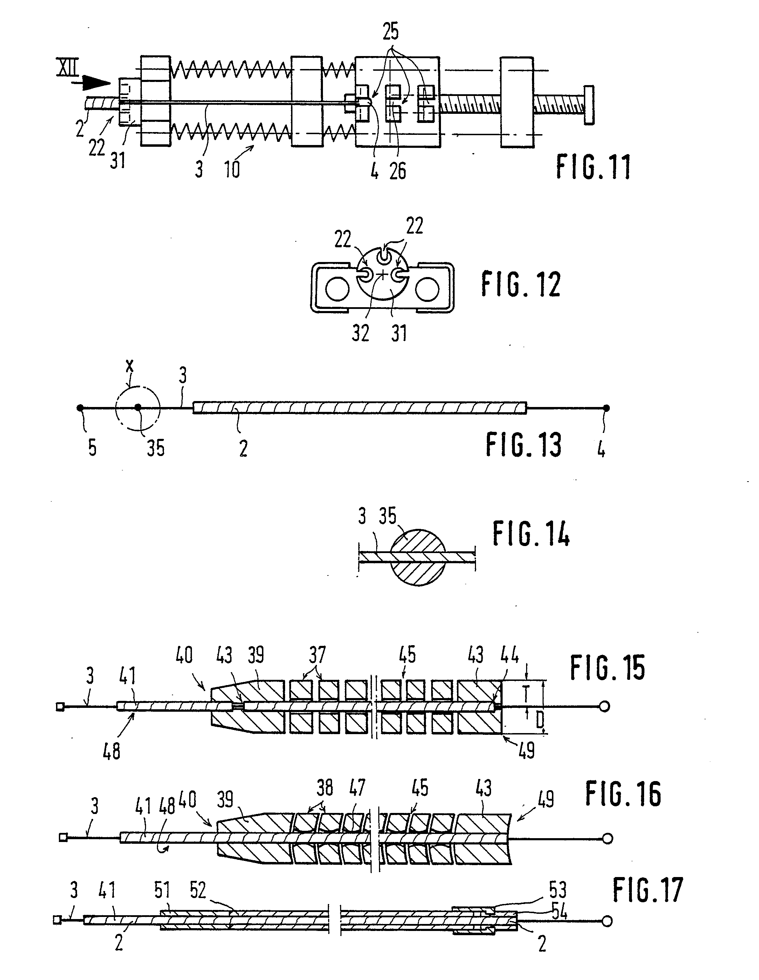

Das zweite Ausführungsbeispiel der Spanneinrichtung 1o gemäß Fig. 11 und 12 unterscheidet sich vom ersten Ausführungsbeispiel lediglich dadurch, daß drei erste Halterungen 22 vorgesehen sind, die an einem Träger 31 ausgebildet sind, der um eine Drehachse 32 drehbar ist, so daß jeweils eine der ersten Halterungen 22 in die gewünschte Arbeitsposition bringbar ist. Der Vorteil dieser Ausgestaltung besteht darin, Sonden unterschiedlicher Abmessungen bzw. Formen an die Spanneinrichtung 1o anschließen zu können. Hierzu sind die ersten Halterungen 22 jeweils so ausgebildet, daß der Mantel der betreffenden Sonde aufgenommen werden kann.

Es sind auch drei zweite Halterungen 25 für das aus dem Mantel 2 hervorragende Ende der Seele 3 der Sonde vorgesehen. Diese drei Halterungen liegen jedoch in Längsrichtung der Spanneinrichtung 1o hintereinander, wodurch es ermöglicht wird, Sonden an die Spanneinrichtung 1o anzuschließen, deren Anschlagteile 4 unterschiedliche Abstände vom Mantel 2 aufweisen. In der Fig. 11 sind drei gebogene Blechwinkel mit Schlitzen 26 gemäß dem ersten Ausführungsbeispiel hintereinander angeordnet. Eine weitere vorteilhafte Ausführungsform für hintereinanderliegende zweite Halterungen 25 kann darin bestehen, einen durchgehenden Schlitz mit Freiräumen (Ausnehmungen) in bestimmten Abständen längs des Schlitzes vorzusehen, in denen die Anschlagteile 4 aufgenommen werden können.

Beim dritten Ausführungsbeispiel gemäß Fig. 13 und 14 besteht die Sonde entsprechend dem ersten Ausführungsbeispiel aus einem durch Wicklungen gebildeten Mantel 2 und einer darin eingeschobenen Seele 3 mit einem vorderen Anschlagteil 5 und einem hinteren Anschlagteil 4 in Kugelform. Darüberhinaus ist in einem Abstand vom Anschlagteil 5 ein weiteres Anschlagteil 35 auf der Seele 3 angeordnet. Der Zweck des Anschlagteils 35 ist folgender.

Löst sich beim Spannen der Sonde aufgrund der Spannkraft das Anschlagteil am vorderen Ende der Seele 3, z.B. aufgrund einer mangelhaften Lötstelle, wird es durch die Vorspannung des Mantels 2 mit Vehemenz weggeschleudert, was theoretisch zu Verletzungen führen könnte. Beim dritten Ausführungsbeispiel wird nicht das Anschlagteil 5, sondern das Anschlagteil 35 beim Spannen belastet, das beim Spannen am Mantel 2 zur Anlage kommt. Löst sich das Anschlagelement 35 beim Spannen unbeabsichtigt, geht es nicht verloren, sondern es wird durch das Spannelement 5 gehalten. Außerdem bleibt die Sonde noch funktionsfähig, weil aufgrund des Vorhandenseins des Anschlagteils 5 die Sonde weiterhin versteift werden kann.

Fig. 14 zeigt die mit X gekennzeichnete Einzelheit in Fig. 13 in vergrößerter Darstellung im Schnitt. Das Anschlagteil 35 ist eine Kugel mit einer Bohrung, die auf die Seele 3 aufgeschoben und verlötet ist.

Im Rahmen der Erfindung ist es auch möglich, mehrere zusätzliche Anschlagteile 35 vorzusehen.

Der Außendurchmesser einer Sonde ist dadurch limitiert, daß einerseits die Seele 3 einen Durchmesser von etwa 1 bis 1,5 mm nicht wesentlich überschreiten darf, da sie sonst zu steif wird, was Schwierigkeiten beim Einführen in eine Stenose mitsichbringt. Außerdem darf insbesondere bei einem aus Wicklungen bestehenden Mantel 2 das Spiel zwischen der Innenwand des Mantels 2 und der Seele 3 nur gering sein, da sich sonst beim Spannen der Sonde der Mantel 2 wellenförmig verformt und bei einem aus Wicklungen bestehenden Mantel 2 sich einzelne Wicklungen übereinanderschieben. Bei einem solchen Ausführungsbeispiel kann auch die Wandstärke des Mantels 2 nicht frei vergrößert werden, da bei einer Stärke der Wicklungen von mehr als 0,6 bis 0,8 mm der Mantel 2 zu steif wird.

Ein größerer Außendurchmesser des Mantels 2 läßt sich ohne Zunahme der Rigidität der nicht versteiften Sonde dadurch erreichen, daß der Mantel 2 aus in seiner Längsrichtung hintereinanderliegenden ringförmigen Gliedern 37,38 gemäß dem vierten und fünften Ausführungsbeispiel nach Fig. 15 und 16 besteht, die beweglich aneinander gehalten sind. Diese Halterung der Glieder 37,38 aneinander kann auf verschiedene Weise, z.B. durch eine für alle Glieder 37,38 gemeinsame Außenhülle (flexibler Schlauch) gebildet werden, die zugleich die Glieder 37,38 und die zwischen ihnen bestehenden Spalte vor Verschmutzung schützt. Von Bedeutung ist, daß die Seele 3 entweder mit dem ersten Glied 39 in Verbindung steht oder mit dem Anschlagteil 5 mittelbar oder unmittelbar gegen das erste Glied 39 ziehbar ist, um die Versteifung zu verwirklichen. Die Versteifung erfolgt durch die Spannung, die bei gegenseitiger Anlage der Glieder 37 bis 39 besteht. Dabei brauchen die Glieder 37 bis 39 nicht ganzflächig aneinander anzuliegen, sondern eine Versteifung ist schon bei punktförmiger Anlage erreicht, bei der die Sonde eine von einer Geraden abweichende Form einnehmen kann.

Bei den zu beschreibenden Ausführungsbeispielen ist der allgemein mit 4o bezeichnete Mantel der Sonde durch einen Kernmantel 41 gebildet, der aus homogenem Material (beispielsweise aus Kunststoff) oder aus aneinanderliegende Wicklungen besteht und auf dem die ringförmigen Glieder 37,38 aufgezogen und aneinander gehalten sind. Das erste Glied 39 und das letzte Glied 43 sind starr, beispielsweise durch Löten,auf dem Kernmantel 41 befestigt. Zum Zweck der Erhöhung der Festigkeit hintergreifen das erste Glied 39 und das letzte Glied 43 den inneren Abschnitt des Kernmantels 41 formschlüssig an den mit 43 und 44 bezeichneten Stellen.

An diesen Stellen 43,44 ist der Kernmantel 41 unterbrochen bzw. verkürzt, wobei die Glieder 39,42 mit Vorsprüngen die vorhandenen Stirnflächen des Kernmantels 41 hinterfassen. Ein Stück vorbestimmter Länge des Kernmantels 41 ragt einführseitig aus dem Glied 39 hervor, womit das Einführen erleichtert wird. Ebenfalls zurerleichterten Einführung ist das erste Glied 39 jeweils konisch zugespitzt. Beim vierten und fünften Ausführungsbeispiel steht nicht nur ein größerer Sondendurchmesser D zur Verfügung, sondern es kann auch eine größere Versteifung erreicht werden, weil größere Radien r vorliegen, von deren Größe das Maß der Versteifung abhängig ist.

Das fünfte Ausführungsbeispiel gemäß Fig. 16 unterscheidet sich vom vierten gemäß Fig. 15 dadurch, daß die Flächen 45, mit denen die Glieder 37,38 aneinanderliegen, sphärische Flächen sind. Hierdurch ist eine stabile Versteifung auch in einer von einer Geraden abweichenden Form der Sonde möglich, beispielsweise einfach oder S-förmig gekrümmt. Außerdem fehlen beim fünften Ausführungsbeispiel die in Fig. 15 mit 43 und 44 bezeichneten Anschlagkanten. Beim fünften Ausführungsbeispiel sind das erste Glied 39 und das letzte Glied 43 durch Löten auf dem aus Wicklungen bestehenden Metallmantel 2 befestigt.

Um die Flexibilität zu vergrößern, ist es vorteilhaft, ein Spiel in den Bohrungen 47 für den Kernmantel 41 vorzusehen. Es ist der Flexibilität insbesondere zuträglich, wenn -wie in Fig. 16 dargestellt-die Bohrungen 47 sich nach außen jeweils erweitern.

Zum Spannen der Sonde gemäß Fig. 15 und 16 wird das dem Einführende 48 abgewandte Ende 49 des Mantels 4o in der ersten Halterung 22 einer Spanneinrichtung 1o aufgenommen. Hier zeigt sich, daß es vorteilhaft ist, erste Halterungen 22 unterschiedlicher Größen an der Spanneinrichtung 1o vorzusehen.

Das sechste Ausführungsbeispiel gemäß Fig. 17 zeigt eine erfindungsgemäß ausgestaltete Sonde, die zum Einführen einer Gallengangprothese 51 in Form eines Kunststoffschlauches vorbereitet ist. Die Prothese 51 ist am Einführende der Sonde auf den Mantel 2 aufgeschoben. Hinter der Prothese 51 befindet sich ein Schubglied 52 in Form eines flexiblen Schlauches, der dazu dient, in der eingeführten Position der Sonde die Prothese 51 in die gewünschte Position zu schieben.

Auf dem hinteren Ende des Schubgliedes 52 ist ein erstes Kupplungsglied 53 befestigt, das an ein zweites Kupplungsglied 54 angekuppelt ist, das auf dem Mantel 2 befestigt ist. In bevorzugter Ausführungsform sind die Kupplungsglieder 53,54 in der Medizintechnik bekannte Luerlock-Anschlußteile, die sich sehr gut eignen.

Es ist der Zweck der Kupplungsglieder 53,54 eine Fixierung zwischen der Sonde (Mantel 2) und dem Schubglied 52 herbeizuführen, so daß eine Relativverschiebung zwischen diesen Teilen nicht erfolgen kann.

Eine Relativverschiebung kann beim Einführen einer Sonde erfolgen, wenn das Einführende beim Einführen auf Widerstand stößt, der die Sonde im Schubglied 52 auswärts zu verschieben sucht. Außerdem führt das Versteifen der Sonde zu einer Kraftkomponente, die dann die Sonde auswärts beaufschlagt, wenn sie in einer eingeführten Position in gekrümmter Stellung versteift wird.Schon durch die Tendenz einer Relativverschiebung wird der behandelnde Arzt erheblich behindert. Eine stattgefundene Relativverschiebung kann sogar zu einem Mißlingen des Eingriffs führen, weil die Prothese 51 eine unpassende Position einnimmt.

Zur Vorbereitung der Sonde wird zunächst das Schubglied 52 vom Einführungsende her aufgeschoben und mit Hilfe der Kupplungsglieder 53,54 an der Sonde festgelegt. Anschließend wird die Prothese 51 vom Einführende her aufgeschoben. Um die Position der Prothese 51 auf der Sonde einstellen zu können, ist es vorteilhaft das Kupplungsglied 54 verstellbar und in der jeweiligen gewünschten Position feststellbar auf der Sonde, im vorliegenden Falle auf dem Mantel 2, anzuordnen. Hierzu kann beispielsweise eine Klemmschraube dienen.

Die erfindungsgemäße Festlegung des Schubgliedes 52 mit Hilfe der aus den Kupplungsgliedern 53,54 bestehenden Kupplung ist auch bei solchen bekannten Sonden vorteilhaft, die nicht erfindungsgemäß versteift werden können und beispielsweise nur aus einer Seele 3 bestehen. Auch beim Einführen solcher Sonden werden -wie schon erwähnt-Kraftkomponenten wirksam, die die Sonde auswärts zu verschieben suchen. Eshalb stellt auch bei solchen Sonden die erfindungsgemäße Ausgestaltung eine bedeutende Erleichterung für den behandelnden Arzt dar. Die Maßnahme, eine Prothese 51 und das Schubglied 52 auf eine eingeführte Sonde vom hinteren Ende her aufzuschieben ist nachteilig. Zum einen deshalb, weil es bei einem Schubglied 52 von etwa 1,5 m Länge einer - schwierigen Handhabung bedarf, zum anderen deshalb, weil die Position der eingeführten Sonde nur dann kontrolliert werden kann, wenn sie immer mit ihrem hinteren Ende aus dem Schubglied 52 herausragt, also etwa 1,5 m länger als an sich erforderlich, bemessen ist. Eine solch lange Sonde ist unhandlich.

### Ansprüche

1. Spanneinrichtung (10) zum Betätigen einer Versteifungs-Sonde (1) zur Verwendung in der Medizin, beispielsweise zu therapeutischen oder diagnostischen Zwecken, insbesondere zur Einführung in den Verdauungs-oder Ausscheidungstrakt, mit drahtförmiger Ausbildung, wobei die Versteifungs-Sonde (1) durch einen aus einem Mantel (2, 40, 41) und einer Draht-Seele (3) bestehenden Bowdenzug gebildet ist und die Draht-Seele (3) am Einführende ein Anschlagteil (5) aufweist, das bei Zug an der Draht-Seele (3) an dem Einführende abgewandten Ende am Mantel (2, 40, 41) oder Anbauteilen desselben zur Anlage kommt, und wobei die Spanneinrichtung (10) umfaßt einen Rahmen (11), welcher zwei kurze Querträger (12, 13) aufweist, die durch wenigstens ein Distanzelement (14) an ihren Enden fest miteinander verbunden sind, eine vorzugsweise lösbar befestigte erste Halterung (22) zum Festhalten des Endes des Mantels des Bowdenzuges, und eine vorzugsweise lösbar befestigte zweite Halterung (25) für das aus dem Ende des Mantels (2) hervorragende Ende der Seele (3), gekennzeichnet durch, zwei Druckstücke (15, 16), die axial verschieblich auf dem Distanzelement (14) angeordnet sind, je eine lange, feste Feder (17) zwischen dem ersten Querträger (12) und dem ersten Druckstück (15) und eine weiche kurze zweite Feder (18) zwischen dem ersten Druckstück (15) und dem zweiten Druckstück (16), und einen mit einem Griffteil (19) ausgerüsteten Gewindebolzen (20), der im wesentlichen entlang der Mittelachse der Einrichtung parallel zu den Führungselementen angeordnet ist, wobei das zweite Druckstück mittig eine durchgehende Gewindebohrung aufweist, durch welche der Gewindebolzen (20) eingeschraubt wird und im zweiten Querträger (13) eine glatte Durchgangsbohrung vorgesehen ist, durch welche das andere Ende des Gewindebolzens (20) verschieblich hindurchgeführt ist, und wobei die erste Halterung (22) zum Festhalten des Endes des Mantels (2) des Bowdenzuges am ersten Querträger (12) und die zweite Halterung (25) für das aus dem Mantel (2) des Bowdenzuges hervorragende Ende der Seele (3) am zweiten Druckstück (16) befestigt ist.

2. Spanneinrichtung nach dem Oberbegriff des Anspruchs 1, wobei ein Druckstück (15) axial verschiebbar angeordnet ist und zwischen dem Druckstück (15) und dem einen Querträger (12) eine Druckfeder (17) angeordnet ist, dadurch gekennzeichnet, daß das Druckstück (15) auf dem Distanzelement (14) axial verschiebbar angeordnet ist und durch einen axial angeordneten, in den anderen Querträger (13) eingeschraubten Gewindebolzen (20) gegen die Druckfedern (17) drückbar ist, wobei die erste Halterung (22) zum Festhalten des Endes des Mantels (2) des Bowdenzuges an dem einen Querträger (12) und die zweite Halterung (25) für das aus dem Mantel (2) des Bowdenzuges hervorragende Ende der Seele (3) am Druckstück (16) befestigt ist.

3. Spanneinrichtung nach Anspruch 1 oder 2, dadurch gekennzeichnet, daß die Druckstücke (15, 16) im wesentlichen die gleiche Ausbildung aufweisen wie die Querträger (12, 13) und über zwei zueinander achsparallele Durchgangsbohrungen auf zwei stangenförmigen Führungselementen (14) verschieblich angeordnet sind.

4. Spanneinrichtung nach einem der Ansprüche 1 bis 3, dadurch gekennzeichnet, daß die erste Halterung (22) bevorzugt an ihrer Oberseite einen mit der Mittelachse des Rahmens (11) gleichgerichteten Schlitz (23) von einer Breite, die etwas größer ist als die Dicke der Seele (3), aufweist und daß das nach der Außenseite des Rahmens (11) weisende Ende des Schlitzes (23) eine Aufweitung (24) aufweist, die etwas größer ist als der äußere Durchmesser des Mantels (2).

5. Spanneinrichtung nach Anspruch 4, dadurch gekennzeichnet, daß die Aufweitung (24) eine konische Ausbildung aufweist.

6. Spanneinrichtung nach Anspruch 4, dadurch gekennzeichnet, daß die Aufweitung (24) eine zum Schlitz (23) koaxiale zylindrische Bohrung mit einem etwas größeren Durchmesser als der Außendurchmesser des Mantels (2) ist.

7. Spanneinrichtung nach einem der Ansprüche 1 bis 6, dadurch gekennzeichnet, daß mehrere erste Halterungen (22) mit unterschiedlich breiten Schlitzen (23) und/oder Aufweitungen (24) vorgesehen sind.

8. Spanneinrichtung nach Anspruch 7, dadurch gekennzeichnet, daß die Halterungen (22) an einem drehbaren Träger (31) angeordnet sind.

9. Spanneinrichtung nach einem der Ansprüche 1 bis 8, dadurch gekennzeichnet, daß die zweite Halterung (25) einen nach oben hin offenen Schlitz (26) aufweist, dessen Weite etwas größer ist als die Dicke der Seele (3).

10. Spanneinrichtung nach den Ansprüchen 1 und 9, dadurch gekennzeichnet, daß die Halterung (25) eine hakenförmige Ausbildung aufweist und aus einem Blech geformt ist, dessen hakenförmiger Teil (27) dem ersten Druckstück (15) zugekehrt ist, wobei die Spitze des Hakens von diesem Teil halbkreisförmig weggebogen ist und der Schlitz (26) in dem Hakenteil (27) eingebracht ist.

11. Spanneinrichtung nach einem der Ansprüche 1 bis 10, dadurch gekennzeichnet, daß in Längsrichtung und/oder Querrichtung mehrere zweite Halterungen (25) hintereinander angeordnet sind.

12. Spanneinrichtung nach Anspruch 1, dadurch gekennzeichnet, daß der Rahmen (11) an seinen beiden Längsseiten abnehmbare U-förmige Abdeckungen (21) aufweist, die das wenigstens die feste Druckfeder (17) tragende Distanzelement (14) im wesentlichen abdeckt.

13. Spanneinrichtung nach einem der Ansprüche 1 bis 12, dadurch gekennzeichnet, daß auf dem Rahmen (11) oder mindestens auf einer der Abdeckungen (21) auf der zu den Druckstücken hinweisenden Seite maßbandähnliche Markierungen (28) vorgesehen sind.

14. Spanneinrichtung nach einem der Ansprüche 1 bis 13, dadurch gekennzeichnet, daß das Druckstück (15) auf der Einleg-Seite der Einrichtung eine mit den Markierungen (28) zusammenwirkende Markierung (29) aufweist.

15. Spanneinrichtung nach einem der Ansprüche 1 bis 14, dadurch gekennzeichnet, daß die Dimensionierung der Sonde und der Einrichtung derart abgestimmt ist, daß der Abstand zwischen den Halterungen (22, 25) und der Abstand zwischen den Mantelteil-und Seelenenden ein leichtes Einziehen des Bowdenzuges von Hand in die Halterungen (22, 25) erlaubt.

16. Spanneinrichtung nach einem der Ansprüche 1 bis 15, dadurch gekennzeichnet, daß als Arretierungseinrichtung die Spanneinrichtung (10) verwendet wird, bei welcher das zweite Druckstück (16) eine an sich bekannte Klemmeinrichtung, wie Klemmschraube, zum Festsetzen des Druckstückes (16) auf den Führungselementen (14) aufweist.

FIG.1

FIG.2

FIG.3

FIG.4

FIG.5

FIG.6

FIG.7

FIG.8

FIG.9

FIG.10

FIG.11

FIG.12

FIG.13

FIG.14

FIG.15

FIG.16

FIG.17